(19) Europäisches Patentamt
European Patent Office
Office européen des brevets

(11) **EP 3 667 671 A2**

(12) **EUROPEAN PATENT APPLICATION**
published in accordance with Art. 153(4) EPC

(43) Date of publication:
**17.06.2020 Bulletin 2020/25**

(51) Int Cl.:
*G16B 20/00* (2019.01)      *G16B 40/00* (2019.01)
*C12Q 1/6834* (2018.01)

(21) Application number: **18843553.1**

(22) Date of filing: **06.08.2018**

(86) International application number:
**PCT/KR2018/008891**

(87) International publication number:
**WO 2019/031785 (14.02.2019 Gazette 2019/07)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **07.08.2017 KR 20170099822**

(71) Applicant: **Yonsei University, University-Industry
Foundation(UIF).
Seoul 03722 (KR)**

(72) Inventors:
• **KIM, Sangwoo**
  **Seoul 04104 (KR)**
• **KIM, Junho**
  **Busan 47193 (KR)**

(74) Representative: **Grünecker Patent- und
Rechtsanwälte
PartG mbB
Leopoldstraße 4
80802 München (DE)**

(54) **METHOD FOR DETECTING VARIATION IN NUCLEOTIDE SEQUENCE ON BASIS OF GENE PANEL AND DEVICE FOR DETECTING VARIATION IN NUCLEOTIDE SEQUENCE USING SAME**

(57)     The present invention provides a method for detection of a mutation in a nucleotide sequence, the method comprising the steps of: obtaining a plurality of target genes for one subject sample by using a gene panel including probes for the plurality of target genes; collecting multiple replicates of nucleotide sequences including nucleotide sequences being identical or non-identical with each of the plurality of target genes by sequencing each of the plurality of target genes in multiple rounds through next generation sequencing (NGS); matching the multiple replicates of nucleotide sequences with reference nucleotide sequences; determining nucleotide sequences unmatched with the reference nucleotide sequences for the plurality of target genes among the multiple replicates of nucleotide sequences; and determining candidates of nucleotide sequence mutations for the plurality of target genes in the subject sample, based on a probability of mutation for a discordant gene locus with the unmatched nucleotide sequences, the probability of mutation being calculated by a computational method according to statistical analysis of unmatched nucleotide sequences.

FIG.1

## Description

## Technical Field

The present invention relates to a gene panel-based method for detection of a mutation in a nucleotide sequence and a device for detection of a mutation in a nucleotide sequence by using the same.

## Background Art

A gene panel is a gene mutation test that analyzes multiple target genes in a panel composed of mutations for target genes and can be utilized in association with the diagnosis or treatment of diseases. Gene mutations can be detected using such gene panels and the next generation sequencing (NGS).

Next generation sequencing is a high-throughput sequencing method that allows the production of massive nucleotide sequence analysis results simultaneously. Together with gene panels, such parallel sequencing at high density can find applications in effectively detecting mutations in nucleotide sequences.

However, even though the same gene panel is employed, the range of variant frequencies in a nucleotide sequence to be detected may vary depending on platforms for next generation sequencing and the analysis methods of nucleotide sequencing data. In addition, the bias generated during polymerase chain reaction for library construction may make it difficult to detect the mutated gene with a variant allele frequency as low as 1 % or less to be masked by false positives appearing on 99% or greater normal genes in next generation sequencing stage.

Therefore, there is a need for a novel method for detection of mutations in nucleotide sequences, which is applicable to a gene panel and allows the detection of low-frequency mutations associated with disease at high sensitivity.

Techniques as a background of the invention have been referred to in order to facilitate understanding of the present disclosure and should not be construed as an admission that the matters described in the technical background of the invention are present in the prior art.

## Detailed Description of the Invention

## Technical Problem

In order to solve the problems with next generation sequencing technology applied to gene panels, the inventors proposed to a method for increasing depths in which identical gene loci are read many times. By using the method, the inventors have aims at increasing the frequencylimit of detection of low-frequency nucleotide sequence mutations, but have recognized that the false positive rates, that is, the errors in analysis for detection are also increased therewith.

Particularly, when a gene panel is applied to investigate nucleotide sequence mutations in association with cancer, the acquisition of accurate information by detecting nucleotide sequence mutations at high sensitivity is important for treating cancer, especially, selecting effective anticancer agents. Cancer may be accompanied with various genomic mutations. Of genomic mutations, somatic mutations may have an influence on the onset or progression of cancer. Such somatic mutations are very difficult to detect, because their allele frequencies are less than 1% in many cases, unlike germline mutations. Moreover, although patients suffer from the same cancer, the patients may have different genomic mutations. For this reason, there is a continued need for a method for detecting a mutation at high sensitivity and accuracy, and particularly a novel mutation detection method applicable to a gene panel.

Meanwhile, the inventors found that the estimation of mutation probability by using replicates allows the reduction of false positives and the detection of low-frequency mutations at high sensitivity. As a result, the present inventors applied the detection technique to a gene panel to develop a novel method for detection of a mutation in a nucleotide sequence by which low-frequency mutations associated with disease can be detected with high sensitivity.

An object of the present disclosure is to provide a method for detection of a mutation in a nucleotide sequence and a device using the same, wherein an analysis error can be reduced to allow the detection of low-frequency nucleotide mutations, by obtaining target genes from one subject sample with probes for target genes provided by a gene panel, sequencing the target genes in multiple rounds to obtain multiple replicates of nucleotide sequences, and providing calibrated probabilities of mutation obtained by the statistical analysis of the multiple replicates of nucleotide sequences.

In addition, the present inventors recognized that new low-frequency mutations associated with disease can be also detected by providing a method of detecting a nucleotide sequence mutation that can be applied to a gene panel and has improved sensitivity.

Another object of the present disclosure is to provide a method for detection of a mutation in a nucleotide sequence and a device using the same, wherein the method comprises matching the nucleotide sequence mutation candidate determined by the detection method of an embodiment of the present disclosure with a nucleotide mutation associated with a disease, to provide information on matching or unmatching between them.

The technical objects of the present disclosure are not limited to the contents exemplified above, and other objects, which are not mentioned above, will be apparent to a person having ordinary skill in the art from the following description.

**Technical Solution**

In order to accomplish the objects, an embodiment of the present disclosure provides a method for detection of a mutation in a nucleotide sequence, the method comprising the steps of: obtaining a plurality of target genes for one subject sample by using a gene panel including probes for the plurality of target genes; collecting multiple replicates of nucleotide sequences including nucleotide sequences being identical or non-identical with each of the plurality of target genes by sequencing each target genes in multiple rounds through next generation sequencing (NGS); matching the plurality of nucleotide sequences of target genes with reference nucleotide sequences; determining nucleotide sequences unmatched with the reference nucleotide sequences for the plurality of target genes among multiple replicates of nucleotide sequences; and determining candidates of nucleotide sequence mutation for target genes in the subject sample, based on a probability of mutation for a gene locus with the unmatched nucleotide sequences in which the probability of mutation is calculated by a calibration method according to statistical analysis of unmatched nucleotide sequences.

According to another embodiment of the present disclosure, the method may further comprise the steps of: obtaining a predetermined nucleotide sequence mutation; and matching the candidates of the nucleotide sequence mutation with the predetermined nucleotide mutation to provide information on matching or un-matching between the candidates of nucleotide sequence mutation and the predetermined nucleotide sequence mutation.

According to another embodiment of present disclosure, the method may further comprise a step of providing information on the candidate of nucleotide sequence mutation and the gene locus thereof, when a given candidate of nucleotide sequence mutation does not match any predetermined nucleotide sequence mutation or a given gene locus of the candidate of nucleotide sequence mutation does not match any predetermined gene loci.

According to another embodiment of the present disclosure, next generation sequencing can be conducted by a plurality of sequencing platforms and the step of collecting multiple replicates of nucleotide sequences can be conducted on the plurality of sequencing platforms wherein nucleotide sequences can be each analyzed on different sequencing platforms.

According to another embodiment of the present disclosure, the step of determining a nucleotide sequence mutation candidate may further comprise a step of identifying association between the nucleotide sequence mutation candidate and the anticancer agent with respect to a therapeutic effect on cancer when the target gene is a cancer-associated gene.

According to another embodiment of the present disclosure, the step of identifying association may comprise identifying a target nucleotide sequence mutation against which an anticancer agent exhibits an anticancer activity.

According to another embodiment of the present disclosure, the step of determining a nucleotide sequence mutation candidate may further comprise a step of determining a nucleotide sequence mutation candidate for the target genes in the subject sample, based on both a probability that a given locus has a true somatic mutation (probability of mutation) and a probability that unmatched nucleotides occurred from a background error (probability of background error) for a gene locus with the unmatched nucleotide sequences, both of the probabilities being calculated by a computational method according to statistical analysis of unmatched nucleotide sequences.

According to another embodiment of the present invention, the probability of background error is estimated for each substitution type of unmatched nucleotide sequence for a given locus on the basis of a background error profile determined according to types of the sequencing platform for the gene panel, allele frequency distribution of background errors per base substitution type, and base call quality scores of the background errors.

According to another embodiment of the present disclosure, the background error profile may further comprise information on nucleotide sequences located ahead of and behind the locus with unmatched nucleotides.

According to another embodiment of the present disclosure, when the panel is designed by Sure-Select, Illumina hybrid-capture or Illumina Amplicon may be utilized as a sequencing platform. In this regard, when sequencing is conducted with Illumina hybrid-capture, the probabilities of background error for base substitution of from C to A and from G to T may be higher than those for other base substitution types.

According to another embodiment of the present disclosure, when sequencing is conducted with Illumina Amplicon, the probabilities of background errors for base substitution of from G to A, from C to T, from T to A, from A to T, from T to C, and from A to G may be higher than those for other base substitution types.

According to another embodiment of the present disclosure, the sequencing panel is an AmpliSeq cancer panel, and IonTorrent Amplicon may be utilized as a sequencing platform. In this regard, when sequencing is conducted with IonTorrent Amplicon, the probabilities of background errors for base substitution types of from G to A, from C to T, from A to C, from T to G, from T to C, and from A to G may be higher than those for other base substitution types.

According to another embodiment of the present disclosure, the step of determining a nucleotide sequence mutation candidate may further comprise a step of determining a nucleotide sequence mutation candidate for the target gene in the subject sample, based on a ratio of the probability of mutation to the probability of background errors for the gene locus with unmatched nucleotide.

According to another embodiment of the present disclosure, the ratio may be calculated according to the following mathematical formula 1:

$$ S_i = \log\left( \frac{\prod_k P(x_i \cap Mut)}{\prod_k P(x_i \cap TE)} \right) $$

(wherein, k is a number of replicates, Xi is BAF (B allele frequency) for an ith gene locus, Mut stands for mutation, and TE stands for a backbround error.)

According to another embodiment of the present disclosure, the target gene may be at least one of the genes ABL1, AKT1, ALK, APC, ATM, BRAF, CDH1, CDKN2A, CSF1R, CTNNB1, EGFR, ERBB2, ERBB4, FBXW7, FGFR1, FGFR2, FGFR3, FLT3, GNA11, GNAQ, GNAS, HNF1A, HRAS, IDH1, IDH2, JAK2, JAK3, KDR, KIT, KRAS, MET, MLH1, MPL, NOTCH1, NPM1, NRAS, PDGFRA, PIK3CA, PTEN, PTPN11, RB1, RET, SMAD4, SMARCB1, SMO, SRC, STK11, TP53, and VHL.

According to another embodiment of the present disclosure, the nucleotide sequence mutation may be a somatic mutation with low variant allele frequency.

According to another embodiment of the present disclosure, the reference nucleotide sequence may be a nucleotide sequence containing no nucleotide sequence mutations for the same target gene as in the subject sample.

According to another embodiment of the present disclosure, the statistical analysis may utilize at least one of the standard deviations and mean values for BAF of the gene locus with unmatched nucleotide of each replicate of nucleotide sequences.

Another object of the present disclosure is to provide a device for detection of a mutation in a nucleotide sequence, the device comprising a processor operably connected to a communication unit, wherein the processor is configured to conduct: acquiring a plurality of target genes for one subject sample by using a gene panel including probes for the plurality of target genes; collecting multiple replicates of nucleotide sequences including nucleotide sequences matched or unmatched with each of the plurality of target genes by sequencing each of the plurality of target genes in multiple rounds through next generation sequencing; matching multiple replicates of nucleotide sequences with reference nucleotide sequences; determining nucleotide sequences unmatched with the reference nucleotide sequences for the plurality of target genes among multiple replicates of nucleotide sequences; and determining candidates of nucleotide sequence mutations for the plurality of target genes in the subject sample, based on a probability of mutation for a gene locus with the unmatched nucleotide, the probability of mutation being calculated by a computational method according to statistical analysis of unmatched nucleotide sequences.

According to another embodiment of the present disclosure, the processor may be configured to conduct matching a nucleotide sequence mutation candidate with the predetermined nucleotide mutation to provide information on accordance or discordance therebetween.

According to another embodiment of the present disclosure, the processor may be configured to provide information on the nucleotide sequence mutation candidate and the gene locus thereof when a given candidate of nucleotide sequence mutation does not match with any predetermined nucleotide sequence mutation or a given gene locus of the candidate of nucleotide sequence mutation does not match with any predetermined gene loci. According to another embodiment of the present disclosure, the processor is configured to determine a nucleotide sequence mutation candidate for the target genes in the subject sample on the basis of both a probability of mutation and a probability of background errors for a gene locus with the unmatched nucleotide sequences, both of the probabilities being calculated by a computational method according to statistical analysis of unmatched nucleotide sequences.

According to another embodiment of the present disclosure, the processor is configured to determine a nucleotide sequence mutation candidate for the target gene in the subject sample, based on a ratio of the probability of mutation to the probability of background errors for the gene locus with the unmatched nucleotides.

According to another embodiment of the present disclosure, the ratio may be calculated according to mathematical formula 1.

## Advantageous Effects

The present disclosure can reduce background errors that can easily mis-interpreted as low-frequency mutation by acquiring a target gene, provided by a target gene, for one subject sample, acquiring multiple replicates of nucleotide sequences through multiple sequencing rounds, and providing a probability of mutation estimated according to the statistical analysis of the nucleotide sequences, whereby the present disclosure has the advantage of detecting low-frequency mutations in a nucleotide sequence. When applied to a gene panel, the detection method with improved sensitivity according to the present disclosure can effectively detect various low-frequency nucleotide sequence mutations associated with diseases.

In the gene panel-based analysis of reads, the method for detection of a mutation in a nucleotide sequence according to an embodiment of the present disclosure can provide a probability of mutation calculated with a computation approach suitably estimated according to sequencing data, irrespective of platforms, whereby a nucleotide sequence mutation can be detected at improved sensitivity.

Based on the improved sensitivity thereof, moreover, the method for detection of a mutation in a nucleotide sequence according to an embodiment of the present disclosure can seek new low-frequency mutations associated with diseases and can provide information thereon in addition to the mutation information supplied by gene panels.

The advantages according to the present disclosure are not limited by the contents exemplified above, and more various effects are included in the specification.

**Brief Description of the Drawings**

FIG. 1 is a block view schematically illustrating the structure of a device for detection of a mutation in a nucleotide sequence according to an embodiment of the present disclosure.

FIG. 2 is a flow diagram illustrating a method for detection of a mutation in a nucleotide sequence according to an embodiment of the present disclosure.

FIGS. 3A and 3B depict multiple replicates of nucleotide sequences for target genes according to the next generation sequencing.

FIG. 3C is a flow chart for illustrating the estimation of a probability of background errors, provided by the method for detection of a nucleotide sequence mutation according to an embodiment of the present disclosure.

FIG. 3D depicts a mutation probability model and a background error probability model, provided by the method for detection of a mutation in a nucleotide sequence according to an embodiment of the present disclosure.

FIG. 4A shows results evaluated by applying the method for detection of a mutation in a nucleotide sequence according to an embodiment of the present disclosure and a conventional detection method to a Illumina SureSelect cancer panel.

FIG. 4B shows results evaluated by applying the method for detection of a mutation in a nucleotide sequence according to an embodiment of the present disclosure and a conventional detection method to an Ion AmpliSeq cancer panel.

FIG. 4C shows validation results of the detected low-frequency mutations by the method for detection of a nucleotide sequence mutation according to an embodiment of the present disclosure.

FIG. 5 shows evaluation results on the sequencing data with multiple replicates by applying the method for detection of a mutation in a nucleotide sequence according to an embodiment of the present disclosure and conventional detection approaches for the analysis of sequencing data with replicates.

FIG. 6a shows measurements for sensitivity and false-positive rate of mutation detection as analyzed by the application of conventional mutation detection methods to Illumina hybrid-capture.

FIG. 6b shows measurements for sensitivity and false-positive rate of mutation detection as analyzed by the application of conventional mutation detection methods to Illumina hybrid-capture.

FIG. 6c shows measurements for sensitivity and false-positive rate of mutation detection as analyzed by the application of conventional mutation detection methods to IonTorrent Amplicon.

**Mode for Carrying Out the Invention**

The advantages and features of the present disclosure, and the manner of achieving them, will be apparent from and elucidated with reference to the embodiments described hereinafter in conjunction with the accompanying drawings. It should be understood, however, that the invention is not limited to the disclosed embodiments, but is capable of many different forms and should not be construed as limited to the embodiments set forth herein. Rather, these embodiments are provided so that this disclosure will be thorough and complete, to fully disclose the scope of the invention to those skilled in the art, and the invention is only defined by the scope of the claims.

The shapes, sizes, ratios, angles, numbers, and the like disclosed in the drawings for describing the embodiments of the present invention are illustrative, and thus the present invention is not limited thereto. Like reference numerals refer to like elements throughout the specification. In the following description, well-known functions or constructions are not described in detail since they would obscure the invention in unnecessary detail. Where "includes", "having", "done", etc. are used in the present specification, other portions may be added unless "only" is used. Unless the context clearly dictates otherwise, Terms in singular form should also be understood to include the plural form.

In interpreting the constituent elements, it should be construed to include the error range even if there is no separate description.

It is to be understood that the features of various embodiments may be partially or entirely coupled or combined with each other and technically various interlocking and driving are possible, and that the embodiments may be practiced independently of each other.

For clarity of interpretation of the present specification, the terms used in this specification will be defined below.

As used herein, the term "target gene" refers to a gene including a genetic region to be sequenced among the entire DNA nucleotide sequence. In this context, the target gene locus may include a specific nucleotide sequence mutation. Accordingly, the target gene can be sequenced and analyzed to seek a nucleotide sequence mutation genetic region therefor.

As used herein, the term "nucleotide sequence mutation" refers to a base substitution in a nucleotide sequence, which may take place due to various factors. For example, a mutation in a nucleotide sequence may be a mutation associated with a disease, particularly, a somatic mutation which results in a disease. However, the nucleotide sequence mutation is not limited to what is described above. By way of example, the nucleotide sequence mutation may further comprise a nucleotide sequence mutation resulting from the contamination of a sample, a germline variant with low variant allele frequency due to a small amount of fetal DNA existing together with maternal DNA in the blood of the mother, and mutations existing in a small amount within a brain cell.

Meanwhile, the somatic mutation may be associated with cancer. Even though suffering from the same cancer, patients may be different from each other in somatic mutation, that is, may have different genomic mutations. Accordingly, the acquisition of accurate information on mutations by detecting mutations of a target gene is important for cancer therapy, particularly, for selecting effective anticancer agents. As such, mutations associated with disease may exits at low frequency in a subject. Hence, detection of low-frequency mutations at high sensitivity is important in diagnosing a disease and furthermore in establishing an effective therapeutic direction.

The term "gene panel", as used herein, refers to a gene mutation test that analyzes multiple target genes to check their mutations. Such a gene panel may be based on next generation sequencing (NGS) and can be used for searching for gene mutations relating to cancer or utilized in association with the diagnosis or therapy of autoimmune disease or hereditary disease. Through a gene panel, a user can perform the analysis of known region for pathogenic mutations and moreover a region to be sought for novel nucleotide sequence mutations. In addition, the user can analyze a plurality of target genes at once through a gene panel. The gene panel may comprise probes having complementary nucleotide sequences to respective target genes and each of the probes can specifically bind to a target genetic region within subject sample DNA through hybridization. For example, a cancer gene panel may comprise a probe for at least one selected from ABL1, AKT1, ALK, APC, ATM, BRAF, CDH1, CDKN2A, CSF1R, CTNNB1, EGFR, ERBB2, ERBB4, FBXW7, FGFR1, FGFR2, FGFR3, FLT3, GNA11, GNAQ, GNAS, HNF1A, HRAS, IDH1, IDH2, JAK2, JAK3, KDR, KIT, KRAS, MET, MLH1, MPL, NOTCH1, NPM1, NRAS, PDGFRA, PIK3CA, PTEN, PTPN11, RB1, RET, SMAD4, SMARCB1, SMO, SRC, STK11, TP53, and VHL genes. Such probes can be used for searching for nucleotide sequence mutations in the target genes. As such, target genes hybridized with the probes can be amplified by PCR to construct a library for sequencing. Ultimately, a nucleotide sequence mutation candidate for a target gene may be identified through next generation sequencing and following analysis.

As used herein, "next generation sequencing" refers to a sequencing technology of genomes which can perform nucleotide sequences at a high speed by treating DNA fragments in a parallel manner. With these features, next generation sequencing is called high-throughput sequencing, massive parallel sequencing, or second-generation sequencing. Various sequencing platforms for next generation sequencing can be used according to purposes. Examples of platforms for next generation sequencing include Roche 454, GS FLX Titanium, Illumina MiSeq, Illumina HiSeq, Illumina Genome Analyzer IIX, Life Technologies SOLiD4, Life Technologies Ion Proton, Complete Genomics, Helicos Biosciences Heliscope, and Pacific Biosciences SMRT. The next generation sequencing technology can be used, together with a gene panel, for detecting mutations in nucleotide sequences. For example, when a gene panel used for detecting a nucleotide sequence mutation associated with a disease is from Illumina, the sequencing platform may be Illumina hybrid-capture or Illumina Amplicon. The sequencing platform may be IonTorrent Amplicon with IonTorrent gene panel for detecting a nucleotide sequence mutation associated with a disease. However, no limitations are imparted thereto.

Even though the same gene panel and sequencing platform are employed, the coverage of detectable allele frequency of nucleotide sequence mutations may vary depending on analysis methods of sequencing data. That is, the detection of low-frequency nucleotide sequence mutations may be dependent on kinds of gene panels and sequencing platforms and finally on analysis methods of sequencing data. Accordingly, there is a need for a novel method that can be applied to a gene panel and can effectively detect various low-frequency nucleotide sequence mutations associated with disease.

As used herein, the term "subject sample" refers to a biological sample obtained from a patient to be identified for a mutation in a nucleotide sequence. The term "reference nucleotide sequence", as used herein, refers to a nucleotide sequence having no mutations for a target gene, in contrast to a subject sample. For example, a subject sample may be a tumor cell having a somatic mutation. Furthermore, sequencing data existing for normal cells may be used for the reference nucleotide sequence, but without limitations thereto.

A nucleotide sequence mutation in a target gene of a subject sample can be detected by comparison with a reference nucleotide sequence for the target gene. For example, a nucleotide sequence sequenced from a subject sample is matched with that from a reference sample. Then, a discordant gene locus at which a unmatch between the nucleotide sequences of the subject sample and the reference sample is formed is selected, and a mutation candidate in the nucleotide sequence of the subject sample may be determined on the basis of a probability of mutation for the discordant gene locus.

As used herein, the term "gene locus" refers to a nucleotide sequence at a specific position among the nucleotide sequences of a sequenced genome, but is not limited thereto, that is, may mean two or more consecutive nucleotide sequences. In addition, the term "probability of mutation" refers to an estimated probability that a discordant gene locus at which a unmatch between a subject sample and a reference sample is formed corresponds to a real nucleotide sequence mutation. The determination of a mutation candidate for a nucleotide sequence in a target gene of a subject sample may be performed, based on probability of mutation and probability of background error, calculated by a computational method according to statistical analysis of multiple replicates of nucleotide sequences, for discordant gene loci of the subject sample.

The term "multiple replicates of nucleotide sequences", as used herein, refers to multiple nucleotide sequences collected by sequencing the same target gene of a subject sample in multiple rounds. In this regard, multiple replicates of nucleotide sequences may be optionally sequenced with different sequencing platforms. Moreover, each of a replicate nucleotide sequences may include multiple reads produced with the increase of the read depth. That is, each of replicate may include the same nucleotide sequence of a target gene. Moreover, multiple replicates of nucleotide sequences may be not identical. Data obtained by singly sequencing a gene in the genome of a sample may include an error of analysis. In light of multiple replicates of nucleotide sequences obtained by sequencing one target gene in multiple rounds, multiple rounds of sequencing provides better detecting accuracy of mutation than a single round of sequencing. In detail, the probability of mutation may vary for each replicate of nucleotide sequences obtained by sequencing the same target gene. For example, if multiple replicates of nucleotide sequences share the same discordant gene loci with the same unmatched nucleotide, this consistency supports higher chance that a given locus has true mutation and thus may have higher probability of mutation than other loci. If only a portion of replicates show the same unmatched nucleotide at the same loci, this discordance supports higher chance that a given locus is affected by background error rather than a true mutation and thus may have lower probability of mutation than other loci

As used herein, the term "BAF" (B allele frequency) refers to a frequency of a specific type of discordant bases (B allele, e.g. A>T) occurring in the total number of sequenced base at a given locus. Accordingly, the probability of mutation may vary depending on BAF for the same discordant gene loci between multiple replicates of nucleotide sequences. For example, a given locus has a consistent BAF between the multiple replicates of nucleotide sequences, this consistency supports higher chance that a given locus has true mutation and thus may have higher probability of mutation than other loci. That is, the probability of mutation for a given discordant gene loci may be correlated with deviations of BAF between the multiple replicates of nucleotide sequences.

As used herein, the term "computational method according to statistical analysis of multiple replicates of nucleotide sequences" refers to a computational method for estimating probability of mutation on the basis of the BAF for one discordant gene locus at which a unmatch exists in each of multiple nucleotide sequences. In detail, the computational method utilizes the standard deviation of BAF to estimate the probability of mutation for discordant gene loci at which un-matches are detected between the multiple nucleotides and the reference sample. In this case, the computational method provides higher probability of mutation for a discordant gene locus with a small standard deviation of BAF than for that with a large standard deviation of BAF for discordant gene loci at which un-matches are detected between the multiple nucleotides and the reference sample. However, no limitations are imparted to the estimation of the probability from the computational method. The computational method may estimate the probability in various manners. For example, the computational method may be a method that provides a lower probability of mutation for a large standard deviation of BAF for a discordant gene locus at which a unmatch is formed between the multiple nucleotide sequences and the reference sample than for a small standard deviation of BAF for a discordant gene locus at which a unmatch is formed between the multiple nucleotide sequences and the reference sample.

Moreover, the method for detection of a nucleotide sequence mutation according to an embodiment of the present disclosure allows the detection of a nucleotide sequence mutation at high accuracy in a manner irrespective of platform types. In detail, the method for detection of a nucleotide sequence mutation according to an embodiment of the present disclosure allows the determination of a nucleotide sequence mutation candidate on the basis of a probability of mutation calculated by a method appropriately calibrated according to sequencing data and a probability of background error. Particularly, the probability of background errors may be an estimated probability of background errors in light of base substitution type. In detail, the probability of background errors is estimated independently per base substitution type, considering the sequencing platform types and a background error profile including base call quality scores thereof. In greater detail, a gene locus with higher base call quality score has higher probability of mutation than that with low base call quality score. The probability of background errors is estimated independently for each substitution type in each replicate, which allows to have independent background error profile per substitution type per replicate considering their different base call quality score. Then, a probability of background errors for each base substitution type is estimated per replicate on the basis of the determined background error profile and combined together. Through such estimation, the method for detection of a nucleotide sequence mutation according to one embodiment of the present disclosure can detect a nucleotide sequence mutation at improved sensitivity even though using multiple sequencing data analyzed by different sequencing platforms.

The nucleotide sequence mutation candidate determined by the method for detection of a nucleotide sequence mutation, which is improved in detection sensitivity by using multiple sequencing data may be matched with a predetermined nucleotide sequence mutation, thereby identifying whether the nucleotide sequence mutation candidate coincides with the predetermined nucleotide sequence mutation. As used herein, the term "predetermined nucleotide sequence mutation" is intended to encompass all the nucleotide sequence mutations that may exist in a target gene. For example, when the gene panel is a cancer gene panel, the predetermined nucleotide sequence mutation may be any mutation in association with cancer.

The determined nucleotide sequence mutation candidate may be a nucleotide sequence mutation that is newly discovered for a specific disease. Accordingly, the determined nucleotide sequence mutation candidate may not match any predetermined nucleotide sequence mutations, and the gene locus of the nucleotide sequence mutation candidate may not match any gene loci of the predetermined nucleotide sequence mutation. In this case, the method for detection of a nucleotide sequence mutation according to an embodiment of the present disclosure may further provide information on the new nucleotide sequence mutation candidate and the gene locus thereof.

In addition, when the subject sample is a tumor cell, the target gene may be a cancer-associated gene. In this regard, anticancer agents effective for the individual subject may vary depending on the nucleotide sequence mutation candidate that the subject sample retains. Accordingly, the method for detection of a nucleotide sequence mutation according to an embodiment of the present disclosure may further provide identifying association between the nucleotide sequence mutation candidate and an anticancer agent with respect to a therapeutic effect on cancer, whereby determination can be made of a target nucleotide sequence mutation against which an anticancer agent exhibits an anticancer activity.

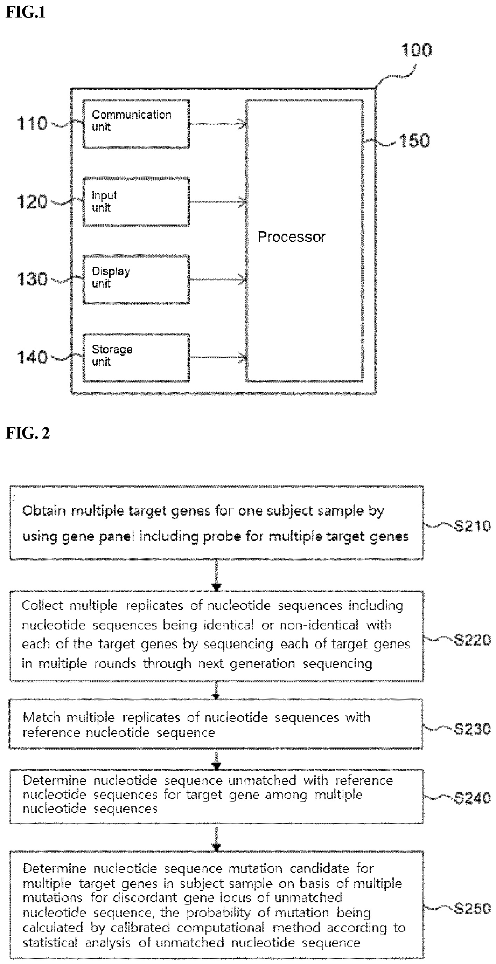

Hereinafter, a device for detection of a mutation in a nucleotide sequence according to an embodiment of the present disclosure is delineated with reference to FIG. 1.

FIG. 1 is a block view schematically illustrating the structure of a device for detection of a mutation in a nucleotide sequence according to an embodiment of the present disclosure. Referring to FIG. 1, a device 100 for detection of a mutation comprises a communication unit 110, an input unit 120, a display 130, a storage unit 140, and a processor 150.

Through the communication unit 110, the nucleotide sequence mutation-detecting device 100 can acquire multiple replicates of nucleotide sequences obtained by sequencing one subject sample multiple times in the next generation sequencing technology. Optionally, the nucleotide sequence mutation-detecting device 100 may acquire a predetermined nucleotide sequence mutation.

Examples of the input unit 120 include a keyboard, a mouse, and a touch screen panel, but are not limited thereto. A user may set up the nucleotide sequence mutation-detecting device 100 and command operations through the input unit 120.

The display 130 can display menus that can be easily set for the nucleotide sequence mutation-detecting device 100 by a user. Furthermore, information about candidates of nucleotide sequence mutations, determined on the basis of the probability of mutation for discordant gene loci, for a target gene in a subject sample, and about accordance or discordance between the determined candidates of nucleotide sequence mutations and the predetermined nucleotide sequence mutations can be provided for a user through the display 130. In addition, when a difference exists between the predetermined nucleotide sequence mutations and the determined candidates of nucleotide sequence mutations, information thereabout can be provided for a user through the display 130. In this regard, the display 130 may be a display device, such as a liquid crystal display device, an organic light-emitting device, etc., and can display menus for a user. In addition, the display 130 may be embodied in various forms or manner within the scope in which the purpose of the present disclosure can be achieved.

The storage unit 140 may store multiple replicates of nucleotide sequences acquired through the communication unit 110. In addition, candidates of nucleotide sequence mutations, determined on the basis of the probability of mutation for discordant gene loci, for a target gene in a subject sample can be stored in the storage unit. Optionally, the storage unit 140 may store information about accordance or discordance between the determined candidates of nucleotide sequence mutations and the predetermined nucleotide sequence mutations. When a difference exists between the predetermined nucleotide sequence mutations and the determined candidates of nucleotide sequence mutations, information about the new candidates of nucleotide sequence mutations and gene loci thereof can be further stored.

The processor 150 performs various orders for operating the nucleotide sequence mutation-detecting device 100 according to an embodiment of the present embodiment. First, the processor 150 is linked to the communication unit 110 and acquires a plurality of target genes for one subject sample through the communication unit 110 by using a gene panel including probes for the plurality of target genes. Then, the processor collects multiple replicates of nucleotide sequences including nucleotide sequences matched or unmatched with each of the plurality of target genes by sequencing each of the plurality of target genes in multiple rounds through next generation sequencing. Subsequently, the processor matches the multiple replicates of nucleotide sequences with reference nucleotide sequences and determines nucleotide sequences unmatched with the reference nucleotide sequences for the plurality of target genes among the multiple replicates of nucleotide sequences. Finally, the processor determines candidates of nucleotide sequence mutations for the plurality of target genes in the subject sample, on the basis of a probability of mutation for a discordant gene locus of the unmatched nucleotide sequences, the probability of mutation being calculated by a computational method according to statistical analysis of unmatched nucleotide sequences.

Below, a detailed description is given of a method for detection of a mutation in a nucleotide sequence according to an embodiment of the present disclosure with reference to FIG. 2.

FIG. 2 is a flow diagram illustrating a method for detection of a mutation in a nucleotide sequence according to an embodiment of the present disclosure.

First, a plurality of target genes for one subject sample is acquired by using a gene panel including probes for the plurality of target genes (S210). In this regard, each of the probes may specifically bind to a target genetic region within a subject sample through hybridization. For example, a cancer gene panel may comprise a probe for at least one selected from ABL1, AKT1, ALK, APC, ATM, BRAF, CDH1, CDKN2A, CSF1R, CTNNB1, EGFR, ERBB2, ERBB4, FBXW7, FGFR1, FGFR2, FGFR3, FLT3, GNA11, GNAQ, GNAS, HNF1A, HRAS, IDH1, IDH2, JAK2, JAK3, KDR, KIT, KRAS, MET, MLH1, MPL, NOTCH1, NPM1, NRAS, PDGFRA, PIK3CA, PTEN, PTPN11, RB1, RET, SMAD4, SMARCB1, SMO, SRC, STK11, TP53, and VHL genes. Target genes hybridized with the probes can be amplified by PCR using such probes to construct a library for sequencing.

Then, multiple replicates of nucleotide sequences including nucleotide sequences matched or unmatched with each of the plurality of target genes are collected by sequencing each of the plurality of target genes in multiple rounds through next generation sequencing (S220). For example, a subject sample may comprise a plurality of reads. These reads are mapped to collect nucleotide sequences for each of the plurality of target genes. In the collecting step (S220), optionally, a matched control sample from the same subject may be sequenced together and served as reference nucleotide sequences. In addition, the collecting step (S220) may be performed using a plurality of sequencing platforms. As a result, multiple replicates of nucleotide sequences can be obtained from different sequencing platforms.

Next, the multiple replicates of nucleotide sequences is matched with reference nucleotide sequences (S230). In the matching step (S220), the reference nucleotide sequences may be matched with each replicate of nucleotide sequences for one target gene. For example, reference nucleotide sequences may be matched with multiple replicates of nucleotide sequences for a target gene according to gene loci in the matching step (S230).

Subsequently, nucleotide sequences unmatched with the reference nucleotide sequences for the plurality of target genes are determined among the multiple replicates of nucleotide sequences (S240). In the unmatched nucleotide sequence-determining step (S240), for example, a search can be made for gene loci discordant with the reference nucleotide sequence in at least one replicate of nucleotide sequences. In this regard, the gene loci discordant with a reference nucleotide sequence for a target gene may be a nucleotide sequence mutation or a background error.

Finally, a nucleotide sequence mutation candidate for the plurality of target genes in the subject sample is determined on the basis of a probability of mutation for a discordant gene locus of the unmatched nucleotide sequences, the probability of mutation being calculated by a computational method according to statistical analysis of unmatched nucleotide sequences (S250). In the step of determining a nucleotide sequence mutation candidate (S250), optionally, a discordant gene locus in the multiple replicates of nucleotide sequence may be determined to be a nucleotide sequence mutation candidate in the subject sample on the basis of both a probability of mutation and a probability of background errors for the discordant gene locus in the unmatched nucleotide sequences. In detail, when a ratio of the probability of mutation to the probability of background errors for a discordant gene locus is a predetermined level or higher, the discordant gene locus may be determined to be a nucleotide sequence mutation candidate in the subject sample. According to another embodiment, the multiple replicates of nucleotide sequences in the step of determining a nucleotide sequence mutation candidate (S250) may be two replicates of nucleotide sequences. In this regard, discordant gene loci in any of the two replicates may be determined to be candidates of nucleotide sequence mutations in the subject sample on the basis of probability resulting from multiplying respective probabilities of mutation for the discordant gene loci of the two replicates. According to various embodiments, the discordant gene locus may be determined to be a background error, irrespective of the probability of mutation, in the step of determining a nucleotide sequence mutation candidate (S250). For example, for a given discordant gene locus, when the mapping quality of the sequence reads is below a predetermined level, when base call quality scores of a majority of bases in a sequenced subject sample is below a predetermined level, or when the fraction of the reads with indel is above a predetermined level, the gene locus of the subject sample may be determined to be a background error irrespective of the probability of mutation. In addition, for a given discordant gene locus, when the fraction of reads that support multiple discordant gene locus is above a predetermined level or when a mutation appears in the matched control data, the gene locus of the subject sample may be determined to be a background error irrespective of the probability of mutation. However, the determination of a gene locus for a background error is not limited thereto.

Furthermore, when the target gene is a cancer-associated gene, association between the nucleotide sequence mutation candidate and the anticancer agent with respect to a therapeutic effect on cancer may be optionally identified in the step of determining a nucleotide sequence mutation candidate (S250). Through the identification, a determination may be made of a target nucleotide sequence mutation against which an anticancer agent exhibits an anticancer activity and furthermore of an anticancer agent effective for the nucleotide sequence mutation candidate.

The nucleotide sequence mutation candidate determined in the step of determining a nucleotide sequence mutation candidate (S250) may be optionally matched with a predetermined nucleotide sequence mutation candidate. As a result, information on the accordance or discordance between the nucleotide sequence mutation candidate and the predetermined nucleotide sequence mutation may be further provided. In this regard, the predetermined nucleotide sequence mutation may be acquired without limitations to any one of the aforementioned nucleotide sequence mutation-detecting steps. Moreover, when a difference is present between the determined nucleotide sequence mutation candidate and the predetermined nucleotide sequence mutation and between the gene loci of the nucleotide sequence mutation candidate and the predetermined nucleotide sequence mutation, information on the nucleotide sequence mutation candidate different from the predetermined nucleotide sequence mutation and on the gene locus thereof may be further provided.

As described above, the method for detection of a nucleotide sequence mutation according to an embodiment of the present invention provides a nucleotide sequence mutation candidate determined in light of various parameters. Accordingly, the method for detection of a nucleotide sequence mutation and the device using the same according to an embodiment of the present disclosure can detect a nucleotide sequence mutation at high sensitivity on the basis of a gene panel and can provide the mutation for a user.

Hereinafter, a detailed description is given of a method for estimating a probability of background errors by using multiple replicates of nucleotide sequences, provided by the nucleotide sequence mutation detecting method according to an embodiment of the present disclosure, for a target gene.

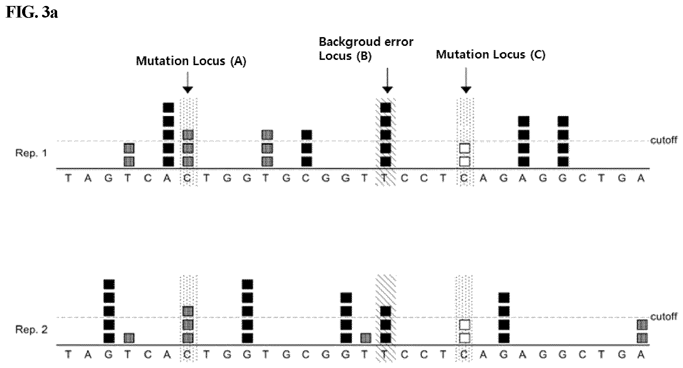

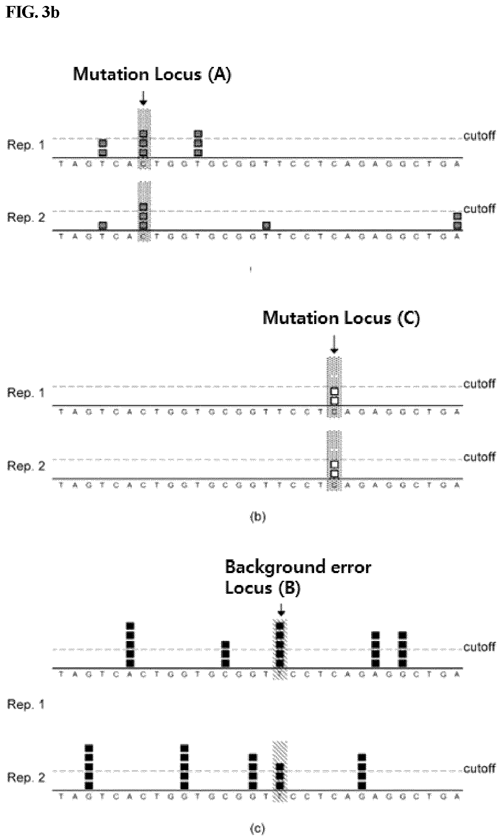

FIGS. 3A and 3B depict multiple replicates of nucleotide sequences for target genes according to the next generation sequencing.

First, with reference to FIG. 3A, there are Rep. 1 and Rep. 2 that are replicates resulting from two rounds of the next generation sequencing for a target gene including (A) to (C) loci. In detail, each square means a degree of discordance with a reference nucleotide for a gene locus that represents BAF. The cutoff value is a criterion for calling a mutation on the basis of a BAF for a gene locus. Conventional methods can determine mutations, based on such cutoff values. Accordingly, a gene locus with a BAF higher than a cutoff value is likely to be described as a nucleotide sequence mutation by conventional methods. However, conventional methods dependent simply on fixed cutoff values result in increased false-positive calls when there are no replicates of sequencing data. As illustrated in FIG. 3A, when multiple replicates of sequencing data (Rep. 1 and Rep. 2) are not considered concurrently, false-positives in Rep. 1 and 5 false-positives in Rep. 2 will be called as mutation. In contrast, when concurrent consideration is taken of both the two sequencing data so as to leave only the concurrently observed loci as mutation candidates, false-positive mutations can be eliminated, except for locus (B) at which a background error has been made, thus greatly contributing to an improvement in accuracy. That is, multiple replicates of sequencing data is needed for improving the detection accuracy of a low-frequency nucleotide sequence mutation.

However, the addition of multiple replicates of sequencing data to conventional cutoff-dependent detection methods is not sufficient for solving the problem with the conventional approaches. For example, high-depth sequencing data for detecting a low-frequency nucleotide sequence mutation frequently contain still many false-positives derived from background errors that beyond the cutoff value repeatedly appear in multiple replicates of sequencing data as in locus (B). In addition, indiscriminate application of a fixed cutoff value may generate many false-negative calls that cannot be detected due to a BAF lower than the cutoff in spite of the existence of real mutations. To solve this problem, flexible determination criteria according to base substitution types are applied on the basis of a probability of mutation and a probability of background errors to determine a mutation candidate in the method for detection of a nucleotide sequence mutation according to an embodiment of the present disclosure.

In detail, with reference to panel (b) of FIG. 3B, locus (C) at which a real mutation is generated for a target gene cannot be a variant call if the analysis is based on the simple cutoff that conventional approaches employ. In the method for detection of a nucleotide sequence mutation according to an embodiment of the present disclosure, however, a very low probability of a background error is assigned to the corresponding locus in the light of the fact that there are almost no observations of loci with that base substitution type. In addition, high probability of mutation is assigned even though this locus shows a low BAF because consistent BAFs are observed in both replicates. In comprehensive consideration of the two factors, the method for detection of a nucleotide sequence mutation according to an embodiment of the present disclosure can determine locus (C) as a mutation. As a result, the method can detect a nucleotide sequence mutation at improve sensitivity.

Turning to panel (c) of FIG. 3b, locus (C) at which a background error is generated for a target gene may be called as a mutation when the analysis is based on the simple cutoff that conventional approaches employ. In contrast, the method for detection of a nucleotide sequence mutation according to an embodiment of the present disclosure can assign a high probability of background errors to the corresponding locus in the light of the fact that there are very frequent observations of loci with that base substitution type. In addition, the method for detection of a nucleotide sequence mutation according to an embodiment of the present disclosure can assign a low probability of mutation to locus (B) even though this locus shows a high BAF in the light of the fact that different BAF values are observed between two replicates. In comprehensive consideration of the two factors, the method for detection of a nucleotide sequence mutation according to an embodiment of the present disclosure can determine locus (B) as a background error. As a result, the method for detection of a nucleotide sequence mutation according to an embodiment of the present disclosure can detect a nucleotide sequence mutation at improved accuracy.

The result of FIG. 3B indicates that multiple replicates of sequencing data (e.g., Rep. 1 and Rep. 2) for one target gene locus must be considered in order to improve the detection accuracy of a nucleotide sequence mutation. Furthermore, gene loci with consistent BAF values (e.g. loci (A) and (C) in Rep.1 and Rep.2) and gene loci with inconsistent BAF values (e.g., locus (B) in Rep. 1 and Rep.2) must be calibrated to be different from each other in terms of probability of mutation and probability of background errors, by considering the base substitution type of corresponding loci.

Accordingly, the method for detection of a nucleotide sequence mutation according to an embodiment of the present disclosure provides a method for estimating a probability of mutation in consideration of BAF values for a gene locus discordant with a reference nucleotide sequence on the basis of multiple replicates for one target gene as in Rep. 1 and Rep. 2. That is, the method for detection of a nucleotide sequence mutation according to an embodiment of the present disclosure may provide a computational method for assigning a high probability of mutation to a discordant locus with a consistent BAF value between replicates (e.g., loci (A) and (C) in Rep. 1 and Rep. 2). In addition, the method for detection of a nucleotide sequence mutation according to an embodiment of the present disclosure may provide a computational method for assigning a relatively low probability of mutation to a discordant locus with an inconsistent BAF value between replicates (e.g., locus (B) in Rep.1 and Rep.2). As a result, the method for detection of a nucleotide sequence mutation according to an embodiment of the present disclosure can provide the detection of a nucleotide sequence mutation at improved accuracy and sensitivity when applied to a gene panel.

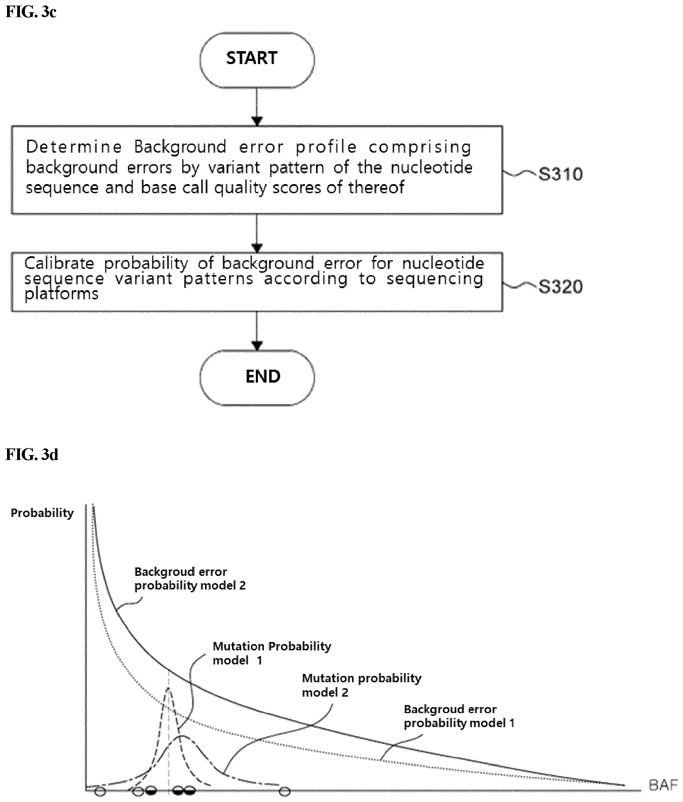

Hereinafter, a method for estimating a probability of background errors for a discordant gene locus, provided by the method for detection of a nucleotide sequence mutation according to an embodiment of the present disclosure, is explained in detail with reference to FIG. 3C.

FIG. 3C is a flow chart for illustrating the estimation of a probability of background errors, provided by the method for detection of a nucleotide sequence mutation according to an embodiment of the present disclosure.

First, a probability of background errors is provided as a estimated value in the light of a base substitution type. In detail, a background error profile comprising background errors by base substitution type and base call quality scores of thereof is determined (S310). In greater detail, a base call quality score may be correlated with an error generated in a sequencing step. For example, a gene locus with a sequencing error may have a low base call quality score while a gene locus with a mutation may have a high base call quality score. However, background error generated in a library construction step prior to a sequencing step may not be dependent on base call quality scores. In the step of determining a background error profile (S310), thus, the background error profile may be determined on the basis of a ratio of background errors generated in a library construction step to the total errors including sequencing errors per base substitution type and it can be different according to sequencing platforms. In the step of determining a background error profile (S310), for example, base call quality scores may be utilized as an index for calibrating sequencing errors in view of base substitution types. In other words, a background error profile of the base substitution type for which a a low base call quality scores are detected and thus expected to have higher burden of sequencing error may be calibrated more to infer true distribution. According to various embodiments, when the sequencing platform is Illumina hybrid-capture, base call quality scores for the base substitution types from C to A and from G to T may be higher than those for the other base substitution types since C to A and G to T background error can be frequently made during the library construction step of Illumina hybrid-capture sequencing. That is, a detection error may be easily made for the base substitution types of from C to A and from G to T which are detected as mutations despite being background errors in Illumina hybrid-capture. When the sequencing platform is Illumina Amplicon, base call quality scores for the base substitution types of from G to A, from C to T, from T to A, from A to T, from T to C, and from A to G may be higher than those for the other substitution types. Furthermore, when the sequencing platform is IonTorrent Amplicon, base call quality scores for the base substitution types of from G to A, from C to T, from A to C, from T to G, from T to C, and from A to G may be higher than those for the other substitution types. As a result, a background error profile comprising background errors by a base substitution type and base call quality scores of thereof is determined in the background error profile determining step (S310). In addition, the background error profile may further comprise information on nucleotide sequences located ahead of and behind the discordant gene locus.

Then, on the basis of the background error profile determined in the background error profile-determining step (S310), the probability of background errors are estimated according to sequencing platforms and base substitution types (S320). For Illumina hybrid-capture, for example, probability of background error for the base substitution types of from C to A and from G to T may be estimated to be higher than those for the other substitution types. For Illumina Amplicon, probability of a background error for the base substitution types of from G to A, from C to T, from T to A, from A to T, from T to C, and from A to G can be estimated to be higher than those for the other substitution types. For IonTorrent Amplicon, probability of a background error for the substitution types of from G to A, from C to A, from A to C, from T to G, from T to C, and from A to G may be estimated to be higher than those for the other substitution types. As a result, a probability of background errors for a discordant gene locus is computed to be a calibrated value in the step of estimating a probability of a background error. Consequently, a nucleotide sequence mutation candidate in a subject sample can be determined, based on a probability of a background error and a probability of mutation, both the probabilities being calculated in consideration of the discordant gene locus.

Hereinafter, a detailed description is given of the step of determining a nucleotide sequence mutation candidate in the subject sample, on the basis of the probability of mutation and the probability of background errors for the discordant gene locus, provided by the method for detection of a mutation in a nucleotide sequence according to an embodiment of the present disclosure.

FIG. 3D depicts a mutation probability model and a background error probability model, provided by the method for detection of a mutation in a nucleotide sequence according to an embodiment of the present disclosure.

In detail, 6 points on the X-axis of the graph represent BAF values for discordant gene loci of replicates, while Y-axis accounts for probability values. In greater detail, BAF values of three replicates of nucleotide sequences for two discordant gene loci, which are produced by three rounds of sequencing for a subject sample, are indicated on X-axis. Mutation probability model 1 and mutation probability model 2 are probability density functions of mutation constructed on the basis of BAF values of the three replicates for the discordant gene loci. In addition, background error probability model 1 and background error probability model 2 are probability density functions of background error, constructed on the basis of the background error profile, for the discordant gene loci accounting for different base substitution types. Referring to the X-axis, a standard deviation of BAF values for three nucleotide sequences corresponding to the three black dots of mutation probability model 1 is smaller than that for three nucleotide sequences corresponding to the three white dots of mutation probability model 2. Accordingly, the probability of mutation for mutation probability model 1 with a low BAF deviation is larger than that for mutation probability model 2 with a relatively large BAF deviation. As a result, the discordant gene locus of mutation probability model 1 can be determined to be a nucleotide sequence mutation candidate in the subject sample because the probability of mutation for mutation probability model 1 with a small BAF deviation is higher than the probability of background errors for background error probability model 1. In contrast, the discordant gene locus of mutation probability model 2 cannot be determined to be a nucleotide sequence mutation candidate in the subject sample because the probability of mutation for mutation probability model 2 with a large BAF deviation is lower than the probability of background errors for background error probability model 2.

Accordingly, the determination of a mutation candidate in a nucleotide sequence of a subject sample may be conducted on the basis of the ratio calculated according to the following mathematical formula 2 set forth in consideration of ratios of the probability of mutation to the probability of background error:

$$S_i = \log\left(\frac{\prod\limits_k P(x_i \cap Mut)}{\prod\limits_k P(x_i \cap TE)}\right)$$

wherein, k is a number of multiple replicates of nucleotide sequences, Xi is BAF (B allele frequency) for an $i^{th}$ gene locus, Mut stands for mutation, and TE stands for a background error. In detail, Si is a log ratio of a multiplication of individual probability values of mutation for k replicates to a multiplication of individual probability values of background error for k replicates. Consequently, when the ratio for a discordant gene locus, calculated by mathematical formula 2, is as high as or higher than a predetermined level, the discordant gene locus may be determined to be a nucleotide sequence mutation candidate in the subject sample. That is, the method for detection of a mutation in a nucleotide sequence and a device for detection of a mutation in a nucleotide sequence using the same according to an embodiment of the present disclosure is based on the ratio, calculated in consideration of various factors, of a probability of mutation to a probability of background errors for a discordant locus at which an unmatch is detected between the multiple replicates of nucleotide sequences and a reference sample and can determine the discordant gene locus as a nucleotide sequence mutation candidate in the subject sample when applied to a gene panel, whereby a nucleotide sequence mutation associated with a disease can be detected at high sensitivity.

## EXAMPLE 1: Evaluation of Method for Detection of Mutation in Inventive Nucleotide Sequence - Cancer Panel

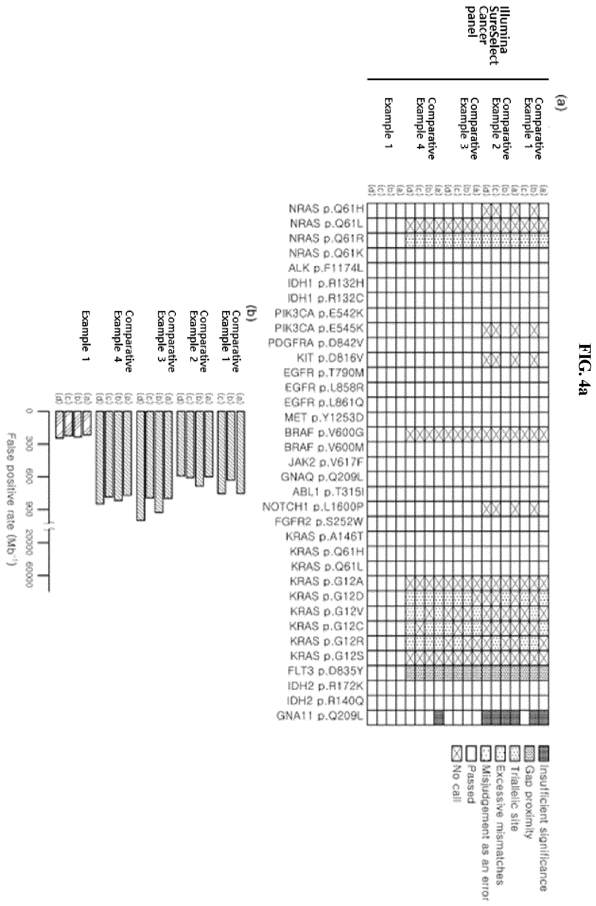

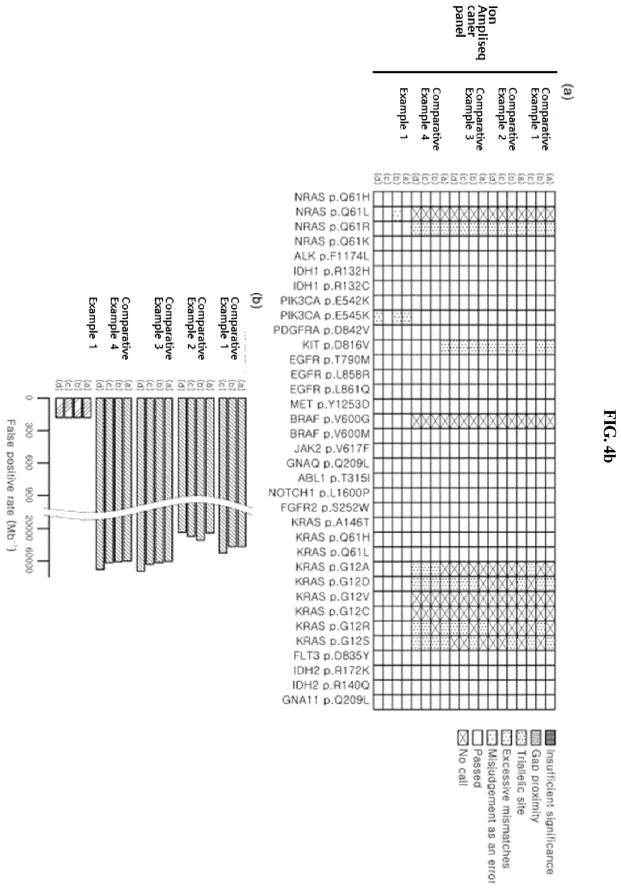

In this Example, evaluation results obtained by applying the method for detection of a mutation in a nucleotide sequence according to an embodiment of the present disclosure and conventional detection methods to a cancer panel are delineated, with reference to FIGS. 4A and 4B. In this evaluation, conventional approaches that utilize multiple replicates include Single, Intersection, BAMerge, and Union. In detail, Single represents a method for detecting a nucleotide sequence mutation by using one replicate. Intersection stands for a detection approach that determine nucleotide sequence mutations per replicate first and get the intersection of mutations between replicates. BAMerge stands for a detection approach in which a nucleotide sequence mutation is determined on the basis of a merged data of replicates. For brevity of description, an evaluation for a cancer panel is given to Embodiment 1 for the application of the method for detection of a mutation in a nucleotide sequence according to an embodiment of the present disclosure, Comparative Embodiment 1 for the application of Single, Comparative Embodiment 2 for the application of Intersection, Comparative Embodiment 3 for the application of BAMerge, and Comparative Embodiment 4 for the application of Union. In the evaluations, reference material with 35 hotspot mutations and wildtype reference material without mutations were employed. In this regard, the mutations included in the reference material include p.Q61H, p.Q61L, p.Q61R, and p.Q61K in NRAS gene, p.F1174L in ALK gene, p.R132H and p.R132C in IDH1 gene, p.E542K and p.E545K in PIK3CA gene, p.D842V in PDGFRA gene, p.D816V in KIT gene, p.T790M, p.L858R, and p.L861Q in EGFR gene, p.Y1253D in MET gene, p.V600G and p.V600M in BRAF gene, p.V617F in JAK2 gene, p.Q209L in GNAQ gene, p.T315I in ABL1 gene, p.S252W in FGFR2 gene, p.A146T, p.Q61H, p.Q61L, p.G12A, p.G12D, p.G12V, p.G12C, p.G12R, and p.G12S in KRAS gene, p.D835Y in FLT3 gene, p.P124L in MEK1/MAP2K1 gene, p.R172K and p.R140Q in IDH2 gene, and p.Q209L in GNA11 gene. Sequencing was conducted in three rounds for the reference material and in one round for the wildtype material. As a result, three replicates of sequencing data (Rep.1, Rep.2, and Rep.3) for the reference material were utilized in the Embodiment and the Comparative Embodiments. In detail, analysis results for combinations of (a) Rep.1 and Rep.2, (b) Rep.1 and Rep.3, (c) of Rep.2 and Rep.3, and (d) Rep.1, Rep.2, and Rep.3 are explained, below.

FIG. 4A shows results evaluated by applying the method for detection of a mutation in a nucleotide sequence according to an embodiment of the present disclosure and a conventional detection method to an Illumina SureSelect cancer panel. FIG. 4B shows results evaluated by applying the method for detection of a mutation in a nucleotide sequence according to an embodiment of the present disclosure and a conventional detection method to an Ion AmpliSeq cancer panel.

With reference to panel (a) of FIG. 4A, evaluation results obtained by applying the detection method of the present disclosure and four conventional detection methods to an Illumina SureSelect cancer panel and Illumina hybrid-capture are illustrated on a matrix. Each cell in the matrix is in a blank space upon the detection of a mutation and is hatched for no detection. In detail, all of the 35 mutations were detected in Embodiment 1 in contrast to Comparative Embodiments 1 to 4. With reference to results of Comparative Embodiments 1 to 4, the Illumina SureSelect cancer panel to which the conventional methods were applied could detect none of the mutations p.Q61L and p.Q61R in NRAS gene, p.V600G in BRAF gene, p.G12A, p.G12D, p.G12V, p.G12C, p.G12R, and p.G12S in KRAS gene, and p.D835Y in FLT3 gene. Particularly, most of the conventional detection methods failed to detect the mutations (no call) or recognized the mutation sites as triallelic sites. Turning to panel (b) of FIG. 4A, the evaluation results of Embodiment 1 were observed to be lower in false-positive rate by two- to three-fold than those of Comparative Embodiments 1 to 4. That is, when applied to the Illumina SureSelect cancer panel, the method for detection of a nucleotide sequence mutation according to an embodiment of the present invention enables mutation detection at high sensitivity with a low false-positive rate.

Referring to panel (a) of FIG. 4B, evaluation results obtained by applying the detection method of the present disclosure and four conventional detection methods to an Ion Ampliseq cancer panel and IonTorrent Amplicon are illustrated on a matrix. Each cell in the matrix is in a blank space upon the detection of a mutation and is hatched for no detection. In detail, the evaluation result of Embodiment 1 include detection of all the mutations except for p.Q61L in NRAS gene due to misjudgment as an error and p.E545K in PIK3CA gene due to excessive unmatches between the site and the reference nucleotide sequence. In contrast, with reference to results of Comparative Examples 1 to 4, the Ion Ampliseq cancer panel to which the conventional detection methods were applied failed to detect mutations p.Q61L and p.Q61R in NRAS gene , p.D816V in KIT gene, p.V600G in BRAF gene, p.G12A, p.G12D, p.G12V, p.G12C, p.G12R, and p.G12S in KRAS gene. Particularly, most of the conventional detection methods failed to detect the mutations (no call) or recognized the mutation sites as triallelic sites. With reference to panel (b) of FIG. 4B, there is as large as a 40-fold difference in false-positive rate between results Embodiment 1 and Comparative Embodiments 1 to 4. That is, the method for detection of a nucleotide sequence mutation according to an embodiment of the present invention enables mutation detection at high sensitivity with a low false-positive rate when applied to the Ion Ampliseq cancer panel as in the Illumina SureSelect cancer panel (FIG. 4A).

Hereinafter, the step of providing information on accordance or discordance between the predetermined nucleotide sequence mutation and the determined candidates of nucleotide sequence mutations, provided by the method for detection of a nucleotide sequence mutation according to an embodiment of the present invention is explained in detail. In this regard, brain disease samples were used as the subject samples. In each sample, analysis was performed for mutations newly discovered against the genes provided by the cancer panel. In greater detail, the analysis utilizes ddPCR (droplet digital PCR) in which each droplet may contain one DNA strand and PCR is carried out for each droplet, thereby identifying whether a mutation is present or absent in the DNA strand contained in each droplet. In addition, ddPCR in this analysis is performed for blank droplets (No template) in order to measure the level of background noise, for droplets containing mutation-free sample DNA as negative controls (Negative), and for droplets that may contain mutant DNA of the brain disease sample.

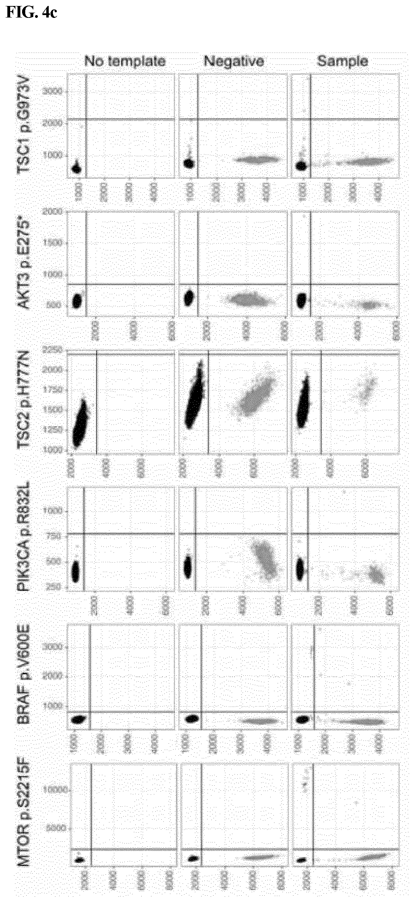

FIG. 4C shows evaluation results of low-frequency mutations detected by the method for detection of a nucleotide sequence mutation according to an embodiment of the present disclosure. In FIG. 4C, each dot means a droplet with the expression of droplets containing no DNA in black, droplets containing normal DNA in green, droplets containing mutant DNA in blue, and droplets containing both normal DNA and mutant DNA in orange.

In detail, the application of the method for detection of a nucleotide sequence mutation according to an embodiment of the present disclosure to a brain disease sample resulted in the discovery of new low-frequency mutations p.G9673V in TSC1 gene, p.E275* in AKT3 gene, p.H777N in TSC2 gene, p.R832L in PIK3CA gene, p.V600E in BRAF gene, and p.S2215F in MTOR gene, which are not detected by conventional approaches. As a result of analysis for the mutations, droplets containing mutant DNA were detected at five among the six variant sites of p.G9673V in TSC1 gene, p.E275* in AKT3 gene, p.H777N in TSC2 gene, p.R832L in PIK3CA gene, p.V600E in BRAF gene, and p.S2215F in MTOR gene, exclusive of p.H777N in TSC2 gene, for the brain disease sample. Accordingly, detection can be made at high sensitivity on the candidates of nucleotide sequence mutations determined by the method for detection of a nucleotide sequence mutation according to an embodiment of the present invention, thereby allowing the detection of new nucleotide sequence mutations different from nucleotide sequence mutations provided by a gene panel. Accordance or discordance between the nucleotide sequence mutation candidate determined by the method for detection of a nucleotide sequence mutation according to an embodiment of the present invention and the predetermined nucleotide sequence mutation can be identified. Furthermore, when the determined nucleotide sequence mutation candidate is a nucleotide sequence mutation newly discovered for a specific disease, information on the new nucleotide sequence mutation candidate for the target gene and the gene locus thereof can be further provided.

Taken together, the results of Example 1 imply that the method for detection of a nucleotide sequence mutation according to an embodiment of the present invention, which can be applied to a gene panel, and a device for detection of a nucleotide sequence mutation using the same can more effectively detect a low-frequency mutation by conducting multiple sequencing rounds for one subject sample and estimating the probability of mutation in consideration of base substitution types. Particularly, the method for detection of a nucleotide sequence mutation according to an embodiment of the present invention can detect nucleotide sequence mutations at high sensitivity and accuracy when applied to Illumina SureSelect and Ion Ampliseq cancer panels. In addition, the method for detection of a nucleotide sequence mutation according to an embodiment of the present invention retains low false-positive rates which lead to a reduction in detection errors. Thus, when applied to various gene panels, the method and device for detection of a nucleotide sequence mutation according to an embodiment of the present invention can provide an analysis for the detection of nucleotide sequence mutations at high sensitivity and accuracy.

## EXAMPLE 2: Evaluation of Inventive Method for Detection of Mutation in Nucleotide Sequence - Multiple Sequencing Platforms

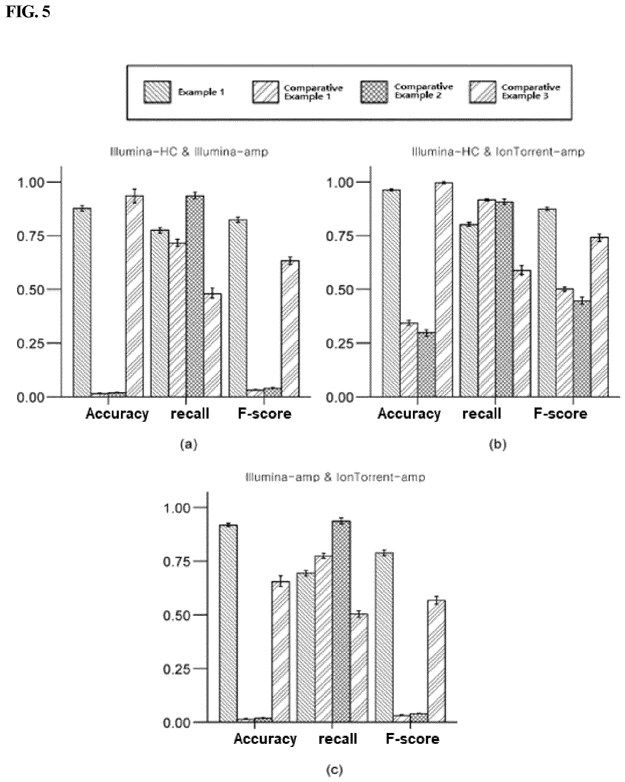

In this Example, evaluation results obtained by applying the method for detection of a mutation in a nucleotide sequence according to an embodiment of the present disclosure and conventional detection methods to multiple sequencing platforms are delineated, with reference to FIG. 5. In this evaluation, conventional approaches include BAMerge, Union, and Intersection. BAMerge and Intersection are the same approaches for detecting a nucleotide sequence mutation as in the evaluation of Example 1. Union stands for a detection approach in which a nucleotide sequence mutation is determined on the basis of a union set of multiple replicates of sequencing data. For brevity of description, an evaluation for a cancer panel is given to Embodiment 1 for the application of the method for detection of a mutation in a nucleotide sequence according to an embodiment of the present disclosure, Comparative Embodiment 1 for the application of BAMerge, Comparative Embodiment 2 for the application of Union, and Comparative Embodiment 3 for the application of Intersection. In Embodiment 1 and Comparative Embodiments 1 to 3, assessment was made of precision, recall, and F-score, which is a balanced measure between precision and recall. FIG. 5 shows results evaluated by applying sequencing data of the method for detection of a mutation in a nucleotide sequence according to an embodiment of the present disclosure and conventional detection approaches to the analysis of sequencing platforms.

Referring to panel (a) of FIG. 5, there are evaluation results obtained by applying sequencing data of the method for detection of a mutation in a nucleotide sequence according to an embodiment of the present disclosure and conventional detection approaches to the analysis with different sequencing platforms of Illumina hybrid-capture and Illumina Amplicon. Embodiment 1 appeared to have the highest precision next to Comparative Embodiment 3. In addition, the F-score in Embodiment 1 was higher than any of Comparative Embodiments 1 to 3 and particularly amounted to about 70 times those in Comparative Embodiments 1 and 2.

Turning to panel (b) of FIG. 5, there are evaluation results obtained for the same target gene by applying sequencing data of the method for detection of a mutation in a nucleotide sequence according to an embodiment of the present disclosure and conventional detection approaches to the analysis with different sequencing platforms of Illumina hybrid-capture and IonTorrent Amplicon. The precision in Embodiment 1 was similar to that in Comparative Embodiment 3, but far higher than those in Comparative Embodiments 1 and 2. The F-score in Embodiment 1 is higher than any of Comparative Examples 1 to 3. With respect to recall, Embodiment was the lowest next to Comparative Embodiment 3.

With reference to panel (c) of FIG. 5, there are evaluation results obtained for the same target gene by applying sequencing data of the method for detection of a mutation in a nucleotide sequence according to an embodiment of the present disclosure and conventional detection approaches to the analysis with different sequencing platforms of Illumina Amplicon and IonTorrent Amplicon. The precision in Embodiment 1 was higher than those in Comparative Embodiments 1 to 3 and particularly amounted to about 60 times those in Comparative Embodiments 1 and 2. In addition, Embodiment 1 was higher in terms of F score and lower in terms of recall than any of Comparative Embodiments 1 to 3.

When applied to a gene panel, as evidenced above, the method for detection of a mutation in a nucleotide sequence according to an embodiment of the present disclosure can determine a nucleotide sequence mutation candidate by providing a probability of mutation calculated with a computation approach suitably calibrated according to sequencing data, irrespective of platforms, whereby a nucleotide sequence mutation can be detected at improved precision.

**COMPARATIVE EXAMPLE 1: Evaluation** of Conventional **Detection Methods for Low-Frequency Mutation**

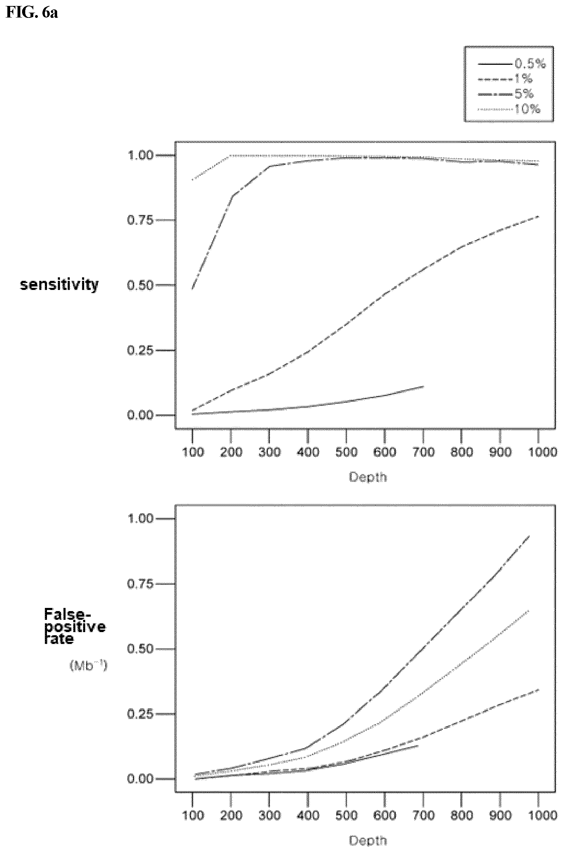

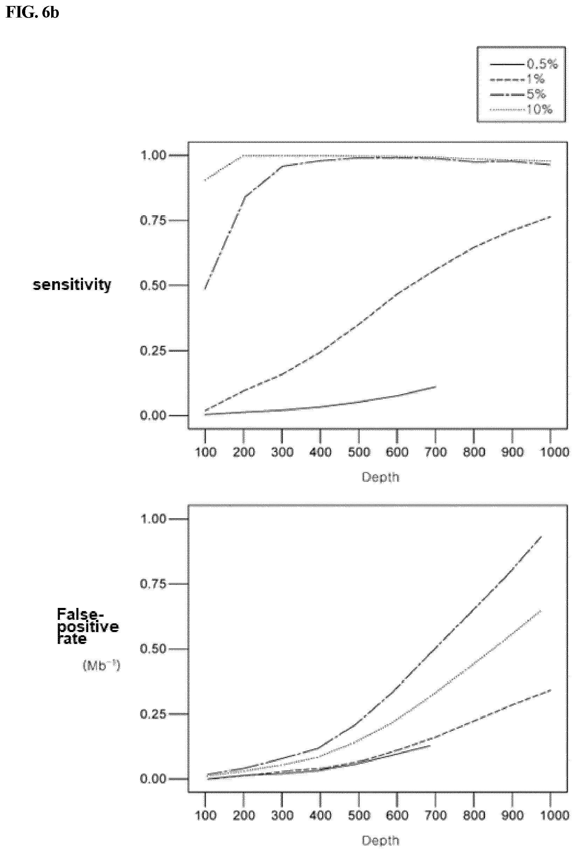

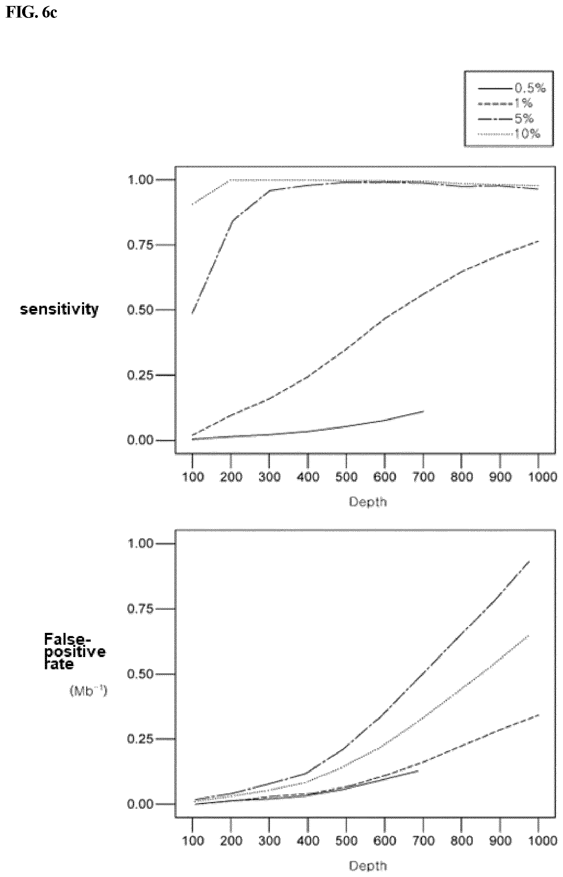

In the comparative example, conventional detection methods for mutations in nucleotide sequences are explained with reference to FIGS. 6a, 6b, and 6c.

FIG. 6a shows measurements for sensitivity and false-positive rate of mutation detection as analyzed by the application of conventional mutation detection methods to Illumina hybrid-capture. FIG. 6b shows measurements for sensitivity and false-positive rate of mutation detection as analyzed by the application of conventional mutation detection methods to Illumina hybrid-capture. FIG. 6c shows measurements for sensitivity and false-positive rate of mutation detection as analyzed by the application of conventional mutation detection methods to IonTorrent Amplicon.

In detail, conventional detection methods used MuTect to detect low-frequency somatic mutations.

For this evaluation, four spike-in samples were employed. In detail, two independent blood samples A and B were mixed to prepare four artificial somatic mutation samples with respective concentrations of 0.5%, 1%, 5%, and 10% of sample B in sample A. In this regard, germline variants in blood sample B acts as a somatic mutations and the four concentrations means BAF of somatic mutations.

With respect to the four spike-in samples, evaluation was made by applying MuTect to the sequencing platforms (Illumina hybrid-capture, Illumina Amplicon, IonTorrent Amplicon).

Referring to FIG. 6A, the sensitivity of detection is lower at 0.5 % of blood sample B in blood sample A than the other concentrations. In addition, false-positive rates are observed to increase with the increasing of the depths. That is, MuTect-applied Illumina hybrid-capture decreased in detection sensitivity for low-frequency mutations.

Referring to FIG. 6B, the sensitivity of detection is lower for 0.5 % of blood sample B in blood sample A than the other concentrations, but the difference in sensitivity among the concentrations is not large, compared to the results from application to Illumina hybrid-capture in FIG. 5A. However, all the samples with the four concentrations greatly increased in false-positive rate with the increasing of depths. In other words, MuTect-applied Illumina hybrid-capture is more prone to detection error when depths are increased in order to detect low-frequency somatic mutations.

Referring to FIG. 6C, the sensitivity of detection is greatly lower for 0.5 % of blood sample B in blood sample A than the other concentrations and false-positive rates are observed to increase with the increasing of the depths.

The results of Comparative Example 1 suggest that all the sequencing platforms to which conventional somatic mutation detection methods are applied are low in detection sensitivity for low-frequency somatic mutations and increase in false-positive rate with the increasing of depths, which leads to the high likelihood of analysis errors. When applied to a gene panel, conventional detection methods for mutations in nucleotide sequences allow the detection of low-frequency nucleotide sequence mutations only at low sensitivity. Hence, the application of conventional detection methods to gene panels may be unsuitable for seeking low-frequency mutations associated disease.

Although the embodiments of the present invention have been described in detail with reference to the accompanying drawings, it is to be understood that the present invention is not limited to those embodiments and various changes and modifications may be made without departing from the scope of the present invention. Therefore, the embodiments disclosed in the present invention are intended to illustrate rather than limit the scope of the present invention, and the scope of the technical idea of the present invention is not limited by these embodiments. Therefore, it should be understood that the above-described embodiments are illustrative in all aspects and not restrictive. The scope of protection of the present invention should be construed according to the following claims, and all technical ideas within the scope of equivalents should be construed as falling within the scope of the present invention.

[Description of Numeral References]

100: Device for detection of nucleotide sequence mutation
110: Communication unit
120: Input unit
130: Display
140: Storage unit
150: Processor
S210: Acquiring step
S220: Collecting step
S230: Matching step
S240: Step of determining unmatched nucleotide sequence
S250: Step of determining nucleotide sequence candidate
S310: Step of determining background error profile
S320: Step of estimating probability of background error

Claims

1. A method for detection of a mutation in a nucleotide sequence, the method comprising the steps of:

obtaining a plurality of target genes for one subject sample by using a gene panel including probes for the plurality of target genes;
collecting multiple replicates of nucleotide sequences including nucleotide sequences being identical or non-identical with each of the target genes by sequencing each of target genes in multiple rounds through next generation sequencing (NGS);
matching the multiple replicates of nucleotide sequences with reference nucleotide sequence;
determining discordant locus of nucleotide sequences unmatched with the reference nucleotide sequence for the plurality of target genes among the multiple replicates of nucleotide sequences; and
determining candidates of nucleotide sequence mutation for the plurality of target genes in the subject sample, based on a probability of mutation for a discordant gene locus of the unmatched nucleotide sequences, where the probability of mutation is calculated by a computational method according to statistical analysis of unmatched nucleotide sequences.

2. The method of claim 1, further comprising the steps of:

obtaining a predetermined nucleotide sequence mutation; and
matching the candidates of nucleotide sequence mutation with the predetermined nucleotide mutation to provide information on accordance or discordance between the candidates of nucleotide sequence mutation and the predetermined nucleotide sequence mutation.

3. The method of claim 2, further comprising a step of providing information on the candidate of nucleotide sequence mutation and the gene locus thereof which does not match any predetermined nucleotide sequence mutation and the gene locus thereto, when the candidate of nucleotide sequence mutation does not match any predetermined nucleotide sequence mutation or the gene locus of the candidate of nucleotide sequence mutation does not match any gene locus of the predetermined nucleotide sequence mutation.

4. The method of claim 1, wherein the step of collecting multiple replicates of nucleotide sequences can be performed by the plurality of sequencing platforms, wherein each of nucleotide sequences being identical or non-identical can be analyzed on different sequencing platforms, and wherein the next generation sequencing can be conducted by a plurality of sequencing platforms.

5. The method of claim 1, wherein the step of determining the candidates of nucleotide sequence mutations further comprises a step of identifying association between the candidates of nucleotide sequence mutations and the anticancer agent with respect to a therapeutic effect on cancer, when the target gene is a cancer-associated gene.

**6.** The method of claim 5, wherein the step of identifying association comprises a step of identifying a target nucleotide sequence mutation to be acted by an anticancer agent.

**7.** The method of claim 1, wherein the step of determining the candidate of nucleotide sequence mutation further comprises a step of determining candidate of a nucleotide sequence mutation for the target genes in the subject sample, based on both a probability that a given locus has a true somatic mutation (probability of mutation) and a probability that unmatched nucleotides occurred from a background error (probability of background error) for a discordant gene locus with the unmatched nucleotide sequences, both of the probabilities being calculated by a computational method according to statistical analysis of unmatched nucleotide sequences.

**8.** The method of claim 7, wherein the probability of background errors is estimated for each substitution type of unmatched nucleotide sequence for a given locus on the basis of a background error profile which is determined according to types of the sequencing platform for the gene panel, allele frequency distribution of background errors per base substitution type, and base call quality score of the background errors.

**9.** The method of claim 8, wherein the background error profile further comprises information on nucleotide sequences located ahead of and behind the discordant gene locus.

**10.** The method of claim 8, wherein, when the type of sequencing platform is an Illumina sequencing platform, the probabilities of background errors for mutation types of from A to G, from T to C, from A to T, from T to A, from C to T, from G to A, from C to A, and from G to T are higher than the probabilities of background errors for other types of the nucleotide sequence mutation.

**11.** The method of claim 8, wherein, when the sequencing platform is an IonTorrent sequencing platform, the probabilities of background error for mutation types of from A to G, from T to C, from C to A, from G to T, from G to A, and from C to T are higher than the probabilities of background errors for other types of the nucleotide sequence mutation.

**12.** The method of claim 7, wherein the step of determining candidate of a nucleotide sequence mutation further comprises a step of determining candidate of a nucleotide sequence mutation for the target gene in the subject sample, on the basis of a ratio of the probability of mutation to the probability of background errors for the discordant gene locus.

**13.** The method of claim 12, wherein the ratio is calculated according to the following mathematical formula 1:

[Mathematical Formula 1]

$$S_i = \log\left(\frac{\prod_k P(x_i \cap Mut)}{\prod_k P(x_i \cap TE)}\right)$$

(wherein, k is a number of replicates, Xi is BAF (B allele frequency) for an $i^{th}$ gene locus, Mut is mutation, and TE is a background error.)

**14.** The method of claim 1, wherein the target gene is at least one of the genes ABL1, AKT1, ALK, APC, ATM, BRAF, CDH1, CDKN2A, CSF1R, CTNNB1, EGFR, ERBB2, ERBB4, FBXW7, FGFR1, FGFR2, FGFR3, FLT3, GNA11, GNAQ, GNAS, HNF1A, HRAS, IDH1, IDH2, JAK2, JAK3, KDR, KIT, KRAS, MET, MLH1, MPL, NOTCH1, NPM1, NRAS, PDGFRA, PIK3CA, PTEN, PTPN11, RB1, RET, SMAD4, SMARCB1, SMO, SRC, STK11, TP53, and VHL

**15.** The method of claim 1, wherein the nucleotide sequence mutation can be a somatic mutation with low variant allele frequency.

**16.** The method of claim 1, wherein the reference nucleotide sequence is a nucleotide sequence containing no nucleotide sequence mutations for the same target gene as in the subject sample.

**17.** The method of claim 1, wherein the statistical analysis utilizes at least one of the standard deviations and mean values for BAF of the discordant gene locus of each replicate of nucleotide sequences.

**18.** A device for detection of a mutation in a nucleotide sequence, the device comprising a processor operably connected to a communication unit, wherein the processor is configured to conduct:

obtaining a plurality of target genes for one subject sample by using a gene panel including probes for the plurality of target genes through the communication unit;
collecting multiple replicates of nucleotide sequences including nucleotide sequences matched or unmatched with each of the plurality of target genes by sequencing each of the plurality of target genes in multiple rounds through next generation sequencing;
matching the multiple replicates of nucleotide sequences with reference nucleotide sequences;

determining nucleotide sequences unmatched with the reference nucleotide sequences for the plurality of target genes among the multiple replicates of nucleotide sequences; and

determining candidates of nucleotide sequence mutation for the plurality of target genes in the subject sample, based on a probability of mutation for a discordant gene locus of the unmatched nucleotide sequences, where the probability of mutation is calculated by a computational method according to statistical analysis of unmatched nucleotide sequences.

19. The device of claim 18, wherein the process is configured to conduct matching the candidate of nucleotide sequence mutation with the predetermined nucleotide mutation to provide information on accordance or discordance therebetween.

20. The device of claim 19, wherein the process is configured to provide information on the candidate of nucleotide sequence mutation and the gene locus thereof which does not match any predetermined nucleotide sequence mutation and the gene locus thereto, when the candidate of nucleotide sequence mutation does not match any predetermined nucleotide sequence mutation or the gene locus of the candidate of nucleotide sequence mutation does not match any gene locus of the predetermined nucleotide sequence mutation.

21. The device of claim 18, wherein the process is configured to determine the candidate of nucleotide sequence mutation further comprises a step of determining candidate of a nucleotide sequence mutation for the target genes in the subject sample, based on both a probability of mutation and a probability of background errors for a discordant gene locus with the unmatched nucleotide sequences, both of the probabilities being calculated by a computational method according to statistical analysis of unmatched nucleotide sequences.

22. The device of claim 21, wherein the process is configured to determine candidate of a nucleotide sequence mutation further comprises a step of determining candidate of a nucleotide sequence mutation or the target gene in the subject sample, on the basis of a ratio of the probability of mutation to the probability of background errors for the discordant gene locus.

23. The device of claim 22, wherein the ratio is calculated according to mathematical formula 1:

[Mathematical Formula 1]

$$S_i = \log\left( \frac{\prod\limits_k P(x_i \cap Mut)}{\prod\limits_k P(x_i \cap TE)} \right)$$

(wherein, k is a number of replicates, Xi is BAF (B allele frequency) for an $i^{th}$ gene locus, Mut is mutation, and TE is a background error.)

**FIG.1**

**FIG. 2**

**FIG. 3a**

**FIG. 3b**

Mutation Locus (A)

Mutation Locus (C)

(b)

Background error
Locus (B)

(c)

**FIG. 3c**

```
                        ┌─────────────┐
                        │    START    │
                        └─────────────┘
                               │
                               ▼
┌──────────────────────────────────────────────────┐
│ Determine Background error profile comprising      │
│ background errors by variant pattern of the       │ ──S310
│ sequence and base call quality scores of thereof   │
└──────────────────────────────────────────────────┘
                               │
                               ▼
┌──────────────────────────────────────────────────┐
│ Calibrate probability of background error for      │
│ sequence variant patterns according to sequencing  │ ──S320
│ platforms                                          │
└──────────────────────────────────────────────────┘
                               │
                               ▼
                        ┌─────────────┐
                        │     END     │
                        └─────────────┘
```

**FIG. 3d**

FIG. 4a

FIG. 4b

(a)

Ion Ampliseq caner panel

Comparative Example 1
Comparative Example 2
Comparative Example 3
Comparative Example 4
Example 1

NRAS p.Q61H
NRAS p.Q61L
NRAS p.Q61R
NRAS p.Q61K
ALK p.F1174L
IDH1 p.R132H
IDH1 p.R132C
PIK3CA p.E542K
PIK3CA p.E545K
PDGFRA p.D842V
KIT p.D816V
EGFR p.T790M
EGFR p.L858R
EGFR p.L861Q
MET p.Y1253D
BRAF p.V600G
BRAF p.V600M
JAK2 p.V617F
GNAQ p.Q209L
ABL1 p.T315I
NOTCH1 p.L1600P
FGFR2 p.S252W
KRAS p.A146T
KRAS p.Q61H
KRAS p.Q61L
KRAS p.G12A
KRAS p.G12D
KRAS p.G12V
KRAS p.G12C
KRAS p.G12R
KRAS p.G12S
FLT3 p.D835Y
IDH2 p.R172K
IDH2 p.R140Q
GNA11 p.Q209L

Insufficient significance
Gap proximity
Triallelic site
Excessive mismatches
Misjudgement as an error
Passed
No call

(b)

Comparative Example 1
Comparative Example 2
Comparative Example 3
Comparative Example 4
Example 1

False positive rate (Mb$^{-1}$)

**FIG. 4c**

**FIG. 5**

(a)

(b)

(c)

**FIG. 6a**

**FIG. 6b**

**FIG. 6c**